# EUROPÄISCHE PATENTSCHRIFT

(11) **EP 2 182 883 B1**
(45) Veröffentlichungstag und Bekanntmachung des Hinweises auf die Patenterteilung: **15.01.2020**
(21) Anmeldenummer: 08828777.6
(22) Anmeldetag: 22.08.2008
(51) Int. Cl.: A61C 19/06, A61N 1/32

(54) **SCHONENDES BEHANDLUNGSGERÄT**
PROTECTIVE TREATMENT APPLIANCE
APPAREIL DE TRAITEMENT INDUISANT MOINS D'INCONFORT

(30) Priorität: 26.08.2007 DE 202007011910 U
(43) Veröffentlichungstag der Anmeldung: 12.05.2010
(73) Patentinhaber: Sardari, David, 80798 München (DE)
(72) Erfinder: MYLIUS, Harald, 84513 Töging (DE)
(74) Vertreter: Gössmann, Christoph Tassilo
(86) Internationale Anmeldenummer: PCT/EP2008/006919
(87) Internationale Veröffentlichungsnummer: WO 2009/027059

(56) Entgegenhaltungen:
- EP-A- 0 279 745
- WO-A-00/56395
- WO-A-2006/072582
- FR-A- 2 699 785
- US-A1- 2001 034 519

## Beschreibung

Bakterien, Vieren und Pilze sind bei Menschen und Tieren und insbesondere bei der Zahnbehandlung schon immer ein Problem gewesen.

In DE 10 2005 000 950 wird ein Behandlungsgerät beschrieben, das eine Vorrichtung aufweist, die ein elektrisches oder elektromagnetisches Feld erzeugt, wobei das elektrische oder elektromagnetische Feld zwischen einer Sonde und dem Körper eines Menschen oder Tieres gebildet wird. Nachteilig an diesem Gerät ist, dass der Patient bei der Entladung, die auftritt, wenn die Sonde dem Körper genähert wird, einen elektrischen Schlag erhält, der für den Patienten unangenehm und schmerzhaft ist.

WO 00/56395 A offenbart ein Behandlungsgerät, das eine Vorrichtung aufweist, die ein elektrisches oder elektromagnetisches Feld erzeugt, wobei das elektrische oder elektromagnetische Feld zwischen einer Sonde und dem Körper eines Menschen oder Tieres oder einem Metall gebildet wird, wobei die Frequenz verringert werden soll, um den Patienten weniger Schmerz zu bereiten.

Aufgabe der Erfindung ist es, den Stand der Technik zu verbessern, insbesondere ein Gerät zur Verfügung zu stellen, bei dem der Patient keinen Stromschlag erhält.

Gegenstand der Erfindung ist ein Behandlungsgerät, das eine Vorrichtung aufweist, die ein elektrisches oder elektromagnetisches Feld erzeugt, sich im wesentlichen in einem Handgriff (11) befindet, der seriell geschaltete Spulen aufweist und eine hohle Glassonde (12) aufweist, die mit zumindest einem elektrisch leitenden Gas gefüllt ist, wobei das elektrische oder elektromagnetische Feld zwischen einer Sonde und dem Körper eines Menschen oder Tieres oder einem Metall gebildet wird, dadurch gekennzeichnet, dass die Feldspannung und Stromstärke innerhalb des elektrischen oder elektromagnetischen Feldes erst bei Annäherung an den Körper oder das Metall, von einer niedrigen Feldspannung und Stromstärke, wobei ein minimaler Strom fließt, wobei es zu einer Entladung kommt, die einen Impuls auslöst, so dass die Stromstärke innerhalb einer Zeiteinheit auf eine höhere, Feldspannung und Stromstärke ansteigt.

Sobald das eingeschalte Behandlungsgerät in die Nähe eines Körpers oder eines Metalls kommt und vorzugsweise dort verbleibt, fließt ein minimaler Strom von vorzugsweise 0,1 µA (der Strom fließt nur solange ein Mindestabstand eingehalten wird), wobei es zu einer Entladung kommt, die einen Impuls auslöst, der die Stromstärke auf eine höhere, vorzugsweise voreingestellte Stromstärke hochfährt. Dies erfolgt innerhalb einer Zeiteinheit von vorzugsweise 0,25 bis zu 10 Sekunden, bevorzugt innerhalb 0,25 bis zu 5 Sekunden, besonders bevorzugt innerhalb 0,5 bis 2 Sekunden, ganz besonders bevorzugt innerhalb einer Sekunde.

Vorzugsweise nach dem Anstieg der Feldspannung und Stromstärke des elektrischen oder elektromagnetischen Feldes fällt diese vorzugsweise innerhalb einer Zeiteinheit wieder ab, wobei sich der Vorgang Anstieg und Abfall der Feldspannung und Stromstärke beliebig oft wiederholen läßt, wobei die Wiederholungshäufigkeit voreingestellt werden kann.

Die erfindungsgemäßen Einstellungen lassen sich innerhalb des Geräts abspeichern, wobei das erfindungsgemäße Behandlungsgerät eine Schnittstelle aufweist, über die alle Einstellungen in dem Gerät von außen veränderbar sind, wobei es sich bei der Schnittstelle vorzugsweise um eine USB, serielle oder parallel Schnittstelle handelt, bevorzugt ist die USB Schnittstelle.

In einer bestimmten bevorzugten Ausführungsform ist die Vorrichtung, die ein elektrisches oder elektromagnetisches Feld erzeugt, so ausgebildet, dass das in dem Feld erzeugte oxidierende Gas, wie Ozon und nascierender Sauerstoff, über eine Gasleitung zu einem Formkörper geführt, der sich an die Anatomie eines Menschen oder Tieres anpaßt. Dieser Formkörper kann jede beliebige Form aufweisen, kann vorzugsweise eine Gebißform, die Form eines Strumpfes oder Schlauches, Glockenform, Handschuhes oder die Form eines ganzen in sich geschlossenen Anzugs aufweisen, um einen Körperteil, wie eine Körperfläche, Wunde, Arm oder einen Fuß zu behandeln. Dieser Formkörper weist eine Zuleitung für das oxidierende Gas und in einer bevorzugten Ausführungsform auch eine Ableitung für das oxidierende Gas auf, so dass ein ständiger Gasaustausch möglich ist. Die Stellen für den Zufuhr und die Abfuhr sind vorzugsweise in einem möglichst großen Abstand von einander anzuordnen. Die Zufuhr und die Abfuhr des oxidierenden Gases erfolgt vorzugsweise über ozongasfeste Membranpumpen, wobei auch Schlauchpumpen möglich ist. Die Absaugstärke ist ein vielfaches des Eingangsstroms in den Formkörper, bzw. des Atemluftstroms in die Lunge, vorzugsweise ist die Absaugstärke um den Faktor 8 stärker, um so ein Ausweichen des Ozongases aus dem Formkörper bzw. ein Einatmen des Ozongases in die Lunge zu vermeiden. Auf diese Weise ist es nicht erforderlich ein Vakuum zu erreichen, welches äußerst zeitaufwendig und schwierig, z.B. am Kiefer, zu erzeugen wäre. Die Absaugstärke ist vorzugsweise um den Faktor 8 stärker als die Zufuhr. Bei einer Unterbrechung des Absaugens vorzugsweise 0,25 bis zu 10 Sekunden, bevorzugt 0,25 bis zu 5 Sekunden, besonders bevorzugt 0,25 bis 2 Sekunden, ganz besonders bevorzugt 0,25 Sekunden oder Reduzierung bis zur Unterbrechung, bricht der Unterdruck zusammen und das oxidierende Gas kann in die kleinsten Hohlräume vordringen.

Das abgesaugte Ozon wird vorzugsweise über Aktivkohle geleitet und als Sauerstoff an die Außenluft abgegeben.

Die Vorteile die mit diesem Formkörper erreichbar sind, sind eine sehr schnelle Wundheilung, keine Karies an den Zähnen bei einer Anwendung von vorzugsweise einmal im Monat. Erweichte Zahnschmelzstellen werden mineralisiert, Parodontitis verhindert oder gestoppt. Anwendungen bei offenen Wunden und Körperflächen oder ganzen Körperteilen, wie Arm oder Bein sollten über 10 Minuten dauern.

Das erfindungsgemäße Behandlungsgerät weist eine Vorrichtung auf, die vorzugsweise ein elektrisches oder elektromagnetisches Feld mit einer Feldspannung von 1.800 V bis 35.000 V mittels einer Spannung von 12 V bis 600 V, einer Stromstärke von 0,1 µA bis 100 µA und einer Frequenz von 10.000 Hz bis 35.000 Hz erzeugt.

Das elektrische oder elektromagnetische Feld wird vorzugsweise mittels einer Spannung von 12 V bis 600 V, bevorzugt einer Spannung von 12 V bis 50 V, besonders bevorzugt von 18 V bis 28 V, vorzugsweise einer Stromstärke von 0,1 µA bis 100 µA, bevorzugt einer Stromstärke von 0,1 µA bis 20 µA, besonders bevorzugt mit einer Stromstärke von 0,8 µA bis 10 µA und vorzugsweise einer Frequenz von 10.000 bis 50.000 Hz, bevorzugt mit einer Frequenz von 25.000 Hz bis 40.000 Hz, besonders bevorzugt mit einer Frequenz von 25.000 Hz bis 38.000 Hz, erzeugt. Die Spannung im elektrischen oder elektromagnetischen Feld beträgt vorzugsweise 1.800 bis 35.000 V, bevorzugt 8.000 bis 18.000 V und besonders bevorzugt 12.000 bis 18.000 V beträgt.

Das erfindungsgemäße Behandlungsgerät ist vorzugsweise zur Anwendung bei Menschen und Tieren gedacht.

Das erfindungsgemäße Behandlungsgerät weist vorzugsweise in einem Handgriff einen Hochspannungstrafo mit Kammerwicklung in Form von seriell geschalteten Spulen, vorzugsweise 12 bis 20, bevorzugt 14 bis 18, besonders bevorzugt 16 auf, die vorzugsweise einen speziellen Kern, vorzugsweise einen Stabkern mit einer Anfangspermeabilität µi von vorzugsweise 350 bis 850, bevorzugt 450 bis 750, besonders bevorzugt 550 bis 650, ganz besonders bevorzugt 600 aufweisen, um eine Feldspannung von 1.800 V bis 35 000 V zu erzeugen. Die oben erwähnte kontrollierte Frequenz von 10.000 Hz bis 50.000 Hz wird mit einer Wiederholungsfrequenz von vorzugsweise 350 Hz bis 500 Hz, bevorzugt 400 Hz bis 480 Hz, besonders bevorzugt 430 Hz bis 460 Hz, ganz besonders bevorzugt 450 Hz oder vorzugsweise 1080 Hz bis 1280 Hz Hz (für die schmerzfreie Kariesbehandlung), bevorzugt 1120 bis 1240 Hz (für die schmerzfreie Kariesbehandlung) aufgebaut. Diese Pulsfrequenz wird über einen Halbleiterschalter, vorzugsweise einen MOS Schalttransistor erzielt, der vorzugsweise im Handgriff plaziert ist, um eine bessere Leistung ohne Induktivität und kapazitive Verluste des Zuführkabels zu erzielen. Diese Pulsart ist ein kurzer Rechtecksimpuls, der eine gedämpfte Schwingung anregt. Die Pulsbreite ist zwischen 1 und 30 µs, bevorzugt 1 bis 13 µs einstellbar, was die Leistung der Erzeugung des atomaren Sauerstoffs von 5% bis 100% bei der höchsten Leistungsstufe steuert.

Der kleine in Kammern gewickelte Hochspannungstrafo ist im Handgriff geschirmt und erzeugt nur minimale elektromagnetische Störungen. Der Gehäuseableitstrom ist dabei mit kleiner 10 µA bei voller Leistung sehr gering. Bei einem Patientenstrom von größer als 80 µA schaltet die Elektronik vorzugsweise automatisch ab. Die Heilungszeit, die Patientenstromüberwachung und Abschaltung wird vorzugsweise über einen Mikrochip geregelt. In klinischen Versuchen wurde eine Heilungszeit von vorzugsweise 40 sec oder länger bei der Zahnbehandlung ermittelt. Dies kann vorzugsweise über einen Signalton angegeben werden.

An dem Handgriff befindet sich eine Sonde, vorzugsweise eine hohle Glassonde, die mit einem elektrisch leitenden Gas gefüllt ist, vorzugsweise mit zumindest einem Edelgas oder beliebigen Edelgasgemischen gefüllt ist. In einer bevorzugten Ausführungsform besteht die Behandlungssonde aus einem Hohlraum aus Glas, der evakuiert oder nicht evakuiert sein kann, in dem sich vorzugsweise ein weiterer Hohlraum aus Glas befindet, der mit Gas gefüllt ist und sich bis zum oberen Ende der Behandlungssonde erstreckt, also in die Spitze der Behandlungssonde übergeht. Die Glassonde weist an ihrem unteren Ende, mit dem sie in dem Handgriff vorzugsweise über eine formschlüssige Klemmverbindung befestigt ist, einen zylinderförmigen Metallverschluss auf. Das obere Ende der Glassonde, also die Spitze ist je nach Applikationsart ausgebildet. Bei Anwendungen im Gebiß weist sie ein spitzes, stumpfes oder auch erweitertes linsenförmiges Ende auf, dabei kann die Spitze selbst eben, konvex oder konkav ausgebildet sein. Für Anwendungen an der Hautoberfläche, wie dermatologische Anwendungen ist ein großes linsenförmiges Ende bevorzugt, um auch größere Flächen behandeln zu können. Eine Sonde um Hämmorrhoiden zu behandeln ist spitz und lang mit einer Erweiterung in der Mitte, Eine Sonde für den Muttermund ist lang und spitz, mit einer leicht abgewinkelten Spitze. Eine Sonde, um Erkrankungen der Kopfhaut oder der Haarfolikel zu behandeln hat die Form eines Kamms, wobei die Zinken des Kammes in diesem Fall U-förmige Glasrohre sind, die mit dem Grundkörper der Sonde so verbunden sind, dass die U-förmigen Röhrchen offen zum Grundkörper der Sonde sind, so dass das Gas ungehindert in der gesamten Sonde zirkulieren kann. Die Zinken betragen 1, 2, 3, 4, 5, 6, 7, 8 etc., wobei 4 Zinken bevorzugt sind. Alle Sonden können prinzipiell auch Kappen, vorzugsweise aus Silicon, tragen, die als Abstandshalter dienen oder in einer Kapillare enden oder in Form eines Bechers ausgebildet sind, der um den Zahn greifen kann. Die Sonde kann auch so ausgebildet sein, dass sie mehr oder weniger formschlüssig ganze Körperteile aufnehmen kann, wie eine Hand, ein Bein, einen Arm oder einen Fuß, etc. Des weiteren können entsprechend gestaltete Glassonden auch am Ohr (Gehörgang), Darmeingang, Scheide, oralen Bereich (z.B. Zunge, Mandeln), sowie an Muskeln, Gelenken, Augen (Lider, Augenabpfel), Naseneingang, Hals- und Rückenwirbel, Fuß oder Hand (Nägel) etc. angewendet werden. Die Glassonde ist vorzugsweise mit zumindest einem leitenden Gas, vorzugsweise einem Edelgas, bei einem Unterdruck von vorzugsweise 0,1 bis 1000 mbar bevorzugt 0,1 bis 500 mbar, besonders bevorzugt 0,1 bis 10 mbar, ganz besonders bevorzugt 2 mbar bis 5, weiter ganz besonders bevorzugt 3 mbar bis 5 bar gefüllt. Als Edelgase werden vorzugsweise beliebige Mischungen aus Edelgasen, vorzugsweise beliebige Mischungen aus Argon und Neon verwendet, wobei Mischungen, die mehr Neon als Argon enthalten, bevorzugt sind, vorzugsweise 0 Vol. % Argon bis 100 Vol. % Neon, bevorzugt 10 Vol.% Argon bis 90 Vol. % Neon, besonders bevorzugt 30 Vol. % Argon bis 70 % Neon, besonders bevorzugt ist eine Mischung aus 30 Vol.% Argon und 70 Vol.% Neon.

In einer weiteren bevorzugten Ausführungsform weist die Behandlungssonde eine stromableitende Vorrichtung zur Erdung auf, diese ist vorzugsweise in der Nähe der Stelle an der Sonde befestigt, die mit dem Körper in Kontakt oder nahe in Kontakt kommt. Bei dieser stromableitenden Vorrichtung handelt es sich vorzugsweise um eine beliebige Vorrichtung, die in der Lage ist Strom zu leiten, dies kann eine Flüssigkeit, ein Gas oder vorzugsweise eine Vorrichtung aus Metall sein, wie bevorzugt ein Draht aus Metall, der vom Glasende der Sonde an ihr entlang zur Masse geführt wird. Vorzugsweise wird der Draht Außen oder auch im Inneren der Sonde entlang zur Masse geführt. Falls der Draht im Inneren der Sonde geführt wird, muss die Sonde doppelwandig sein. Die stromableitende Vorrichtung, wie zum Beispiel eine stromleitende Flüssigkeit oder stromleitendes Gas, vorzugsweise ein Draht aus Metall, kann sich auch vorzugsweise in einem an der Behandlungssonde befestigtem Rohr aus vorzugsweise Kunststoff, Metall oder bevorzugt Glas befinden. Der Draht ist vorzugsweise in der Nähe der Stelle an der Sonde befestigt, die mit dem Körper in Kontakt oder nahe in Kontakt kommt, wobei zwischen der Ableitung, also dem Draht und der Glassonde ein Luftraum ist, vorzugsweise wird er am oberen Ende der Behandlungssonde befestigt, das mit dem Körper in Kontakt kommt, wobei bei einem konkaven Sondenende er auch vorzugsweise in der Kavität befestigt sein kann, bei einem spitzen Sondenende vorzugsweise irgendwo entlang der Sondenspitze befestigt sein kann und bei einem linsenförmigen Sondenende vorzugsweise am Linsenrand befestigt ist. Die Befestigung der stromableitenden Vorrichtung erfolgt vorzugsweise, indem der Draht am oberen Ende der Behandlungssonde in das Glas eingeschmolzen ist, angeklebt ist oder vorzugsweise über eine Klemmverbindung befestigt ist, die über die Spitze der Behandlungssonde gezogen wird. Diese Klemmverbindung kann als Ring ausgebildet sein, der aus Metall oder vorzugsweise einem Kunststoff ausgebildet ist, der vorzugsweise starr oder bevorzugt flexibel sein kann und bevorzugt ein Siliconelastomer ist. Überraschender Weise wurde festgestellt, dass nun auch Patienten, die besonders schmerzempfindlich sind, bei Behandlungssonden, die die stromableitende Vorrichtung zur Erdung aufweisen, nun überhaupt keinen Schmerz mehr empfinden.

Die Quelle für das oxidierende Gas ist derart aufgebaut, dass das Behandlungsgerät das oxidierende Gas direkt an einer Glassonde erzeugt, vorzugsweise aus dem umgebenden Luftsauerstoff oder auch aus reinem Sauerstoff, wobei die Behandlungssonde an dem Handgriff angebracht ist, indem nach dem Prinzip der stillen elektrischen Gasentladung ein elektrisches Feld zwischen zwei Polen, hier die Sonde und ein Patient, erzeugt wird, die durch einen Isolator, nämlich Luft getrennt ist. Ab einer Grenzfeldstärke werden in dem elektrischen Feld durch andauernde Entladevorgänge Elektronen erzeugt, die den in der Umgebungsluft enthaltenen Sauerstoff in ionisierten Sauerstoff, in Radikale spaltet, wie hochreaktive Substanzen (Sauerstoff im statu nascendi) wie atomarer Sauerstoff , der hauptsächlich gebildet wird, Hydroxylionen, Ozon und andere gebildet. Nach dem Anlegen einer Wechselspannung mit bis zu 35 000 Hz entsteht zwischen der Elektrode, der Glassonde und dem Patienten ein Entladevorgang und - hiermit verknüpft - ein elektrisches Feld mit hoher Elektronendichte. Die Radikalen entstehen, indem Moleküle der Luft von Elektronen getroffen werden und hierbei ein erhöhtes Energieniveau erreichen. Der atomare Sauerstoff, der hochreaktiv ist, reagiert mit den Keimen der Applikationsstelle, dabei entsteht auch etwas Ozon, das jedoch weit unter der MAK (maximale Arbeitsplatzkonzentration) Grenze von 0,1 ppm liegt, nämlich nur bei 18 % des MAK Werts. Überraschend ist, dass gerade im erfindungsgemäßen Bereich der Stromstärke und der Frequenz besonders viel atomarer Sauerstoff erzeugt wird. Der atomare Sauerstoff wird sowohl vor der Ozonbildung als auch beim Zerfall des Ozons gebildet. Das beim erfindungsgemäßen Behandlungsgerät gebildete Ozon zerfällt, soweit es vom Gewebe resorbiert wird, im wässerigen Milieu, insbesondere bei der Zahnbehandlung, das heißt in der interzellulären Substanz innerhalb von ca. 10 Minuten auf den halben Wert seiner ursprünglichen Konzentration.

Das erfindungsgemäße Behandlungsgerät ist auf Grund seiner niedrigen Stromstärke und hohen Bildung an atomaren Sauerstoff besonders geeignet zur Behandlung von Tieren und Menschen an der Hautoberfläche, um Keime zu beseitigen, die Durchblutung zu fördern und damit auch den Lymphabfluß zu erhöhen. Somit ist es besonders geeignet zur Behandlung von infizierten Wunden, Dekupitus, Nagelbettentzündungen, Akne, Kopfschmerzen, Migräne, Hämatomen, Psoriasis, Neurodermitis, Muskel- und Gelenkentzündungen, allgemeine Sportverletzungen, allgemeine Dermatologie, Gynäkologie, Augenheilkunde, Tumore, Dermatitis, infektiöse Hauterkrankungen, Erkrankungen der Haarfollikel und Talgdrüsen, Verhornungsstörungen, Blasen-bildende Erkrankungen der Haut, Papulo-squamöse Erkrankungen der Haut, Warzen und Zahnheilkunde.

Das erfindungsgemäße Behandlungsgerät als Zahnbehandlungsgerät tötet alle Keime an der Applikationsstelle innerhalb von ca. 40 Sekunden.

Bevorzugt wird das erfindungsgemäße Zahnbehandlungsgerät bei folgenden Anwendungsgebieten eingesetzt.
1. Gingivitis
2. Parodontitis
3. Stomatitis (incl. Mykosen)
4. Aphthe(n)
5. Herpes (lapialis)
6. Peri-implantitis
7. Dentitio difficilis
8. Kavität (nach Präperation)
9. Hypersensibilität
10. Caries superficialis (Fissurenkaries, Zahnhalskaries nach Präperation)
11. Caries media
12. Caries profunda (Restkaries)
13. Pulpa-Trepanation/-hyperämie
14. Wurzelkanal - Behandlung (Final - Maßnahme)
15. Kronenstümpfe (vor dem Zementieren der Krone)
16. Extractionswunde(n) (Koagula - Stabilisierung)
17. Koagulaphatie (Alviolitis)
18. Sickerblutung(en)
19. Fräskanal(vor Implantat inkorporation)
20. Wundgebiet (intra-/ post operationem)
21. Furkationsbefall
22. Fistel
23. Neuralgien
24. Myoarthropathien (incl. Parafunktionen)

Besonders überraschend ist, dass mit dem erfindungsgemäßen Behandlungsgerät eine schmerzfreie Kariesbehandlung möglich ist. Die Exkavierung der Karies mit mechanischen Instrumenten kann damit auf ein Minimum reduziert werden, da durch die absolute Keimabtötung kaum mehr Zahnmaterial abgetragen werden muss. Eine Pulpaverletzung, beziehungsweise eine Verkeimung, denen heute eine Wurzelbehandlung nachfolgt, mit dem Verlust der Vitalität des Zahns, lassen sich, auf Grund der Keimabtötung mit dem erfindungsgemäßen Behandlungsgerät, mit dem Erhalt der Vitalität des Zahnes, erfolgreich behandeln. Besonders überraschend ist, dass beherdete Zähne (Zähne unter Eiter) sich, nach dem sie aus dem Kiefer entfernt worden sind und nachdem sie und die Stelle, aus der der beherdete Zahn entnommen worden ist, mit dem erfindungsgemäßen Behandlungsgerät behandelt worden ist und so der Zahn und der Kiefer keimfrei gemacht worden sind, wieder implantiert werden und nach spätestens 10 Tagen wieder bissfest im Kiefer festgewachsen sind, so dass Implantate überflüssig geworden sind. Mit der Behandlung mit dem erfindungsgemäßen Behandlungsgerät lassen sich in der Regel chirurgische Eingriffe und die Gabe von Antibiotika vermeiden.

Eine weitere bevorzugte Ausführungsform des erfindungsgemäßen Behandlungsgerät ist ein Gerät, bei dem die Vorrichtung, die ein elektromagnetisches Feld bildet, jeweils an den zwei Enden eines offenen kreisförmigen Bügels, ähnlich einem Kopfhörer, angeordnet ist, die in einer Glassonde endet, um das elektrische oder elektromagnetische Feld vorzugsweise an der Kiefergelenkmuskulatur zu erzeugen. Mit diesem Gerät werden zentrale neurogene Fixierungen behoben.

Das Wirkprinzip der Vorrichtung zielt ab auf die pathologischen Vorgänge in der verspannten Muskulatur und Myogelose. Es bildet die therapeutische Grundlage für eine weitgehende Normalisierung der pathologischen Zustände.

Utilisierte Mengen an atomaren Sauerstoff stimulieren die Bildung von Glutathionperoxidase, welche die Glykolyse aktiviert.

Durch das erfindungsgemäße Prinzip kommt es zu einer Sauerstoffsättigung am Hämoglobin (ausreichendes Sauerstoffangebot für eine aerobe Muskelkontraktion).

Die rheologischen Eigenschaften des Blutes nehmen zu. Der Überschuss freier Radikale wird eliminiert.

Gleichzeitig stimulieren Magnetfeld-Impulse die Muskulatur. Eine Durchblutungsanregung und Temperaturerhöhung ist die Folge. Dies führt zu einem beschleunigten Lymphabfluss.

Mit der erfindungsgemäße Vorrichtung wird selbst hochgradig verspannte Kiefergelenkmuskulatur relaxiert. Diese bevorzugte Ausführungsform der erfindungsgemäßen Vorrichtung eignet sich besonders zur Prophylaxe:
1. Für eine exakte, fehlerfreie Bissnahme.
2. Zur Neutralisation von Fehlstellungen.
3. Vermeidung von erschwerter Behandlung aufgrund eingeschränkter Mundöffnung.
4. Vermeidung von Folgekrankheiten, die aus einem Fehlbiss herrühren.
5. Vermeidung von Überdehnungen aufgrund längerer Behandlungen und zur Therapie von:
6. Schmerzbefreiung und Entkrampfung
7. Migräne (als Folge einer Fehlstellung des Kiefergelenkes).
8. Beschwerden der Wirbelsäule (aufgrund einer Fehlstellung des Kiefergelenkes)
9. Nacht- bzw. Tagknirschen
10. Arthrose des Kiefergelenks (Kiefergelenkknacken)
11. Kiefersperre (Bisssperre)

### Wirksamkeitstest

Das Ausgangsmaterial bestand durchweg aus kariösen Zähnen, die nach der Extraktion in ein Transportgefäß mit physiologischer Kochsalzlösung gelegt wurden. Es wurden folgende Proben mittels Stahlfräser (Rosenbohrer 1,9 mm Durchmesser) beziehungsweise mit diamantierten Separierscheiben hergestellt:
Gruppe 1 gefrästes, kariöses Zahnmaterial (Bohrspäne);
Gruppe 2: Zahnsegmente, präpariert bis zum Grund der Erweichungszone;
Gruppe 3: Zahnsegmente, präpariert über die Erweichungszone hinaus bis in das verfärbte, sondenharte Dentin.

Alle Proben wurden nach ihrer Bearbeitung für 24 Stunden (jeweils separat) in steriler isotoner Natriumchloridlösung pro injektionem im Brutschrank bei 35 Grad Celsius aufbewahrt, dann kräftig geschüttelt und anschließend in jeweils eine Petrischale mit Columbia-Agar (Blutagar mit 5 Prozent Hammelblutzusatz) geschüttet und dann die Bakterien suspension gleichmäßig über den gesamten Nährboden verteilt. Die Bebrütung erfolgte über 100 Stunden bei ebenfalls 35 Grad Celsius.

Alle Gruppen wurden mit dem erfindungsgemäßen Zahnbehandlungsgerät 40 Sekunden behandelt. Danach war die behandelte Petrischale der Gruppe 1 - 3 keimfrei.

### Figuren

**Figur 1**

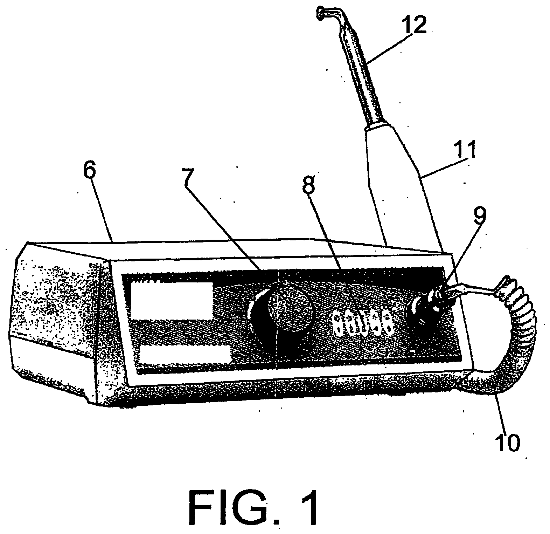

   Figur 1 zeigt eine bevorzugte Ausführungsform des erfindungsgemäßen Behandlungsgeräts.
**Figur 2**

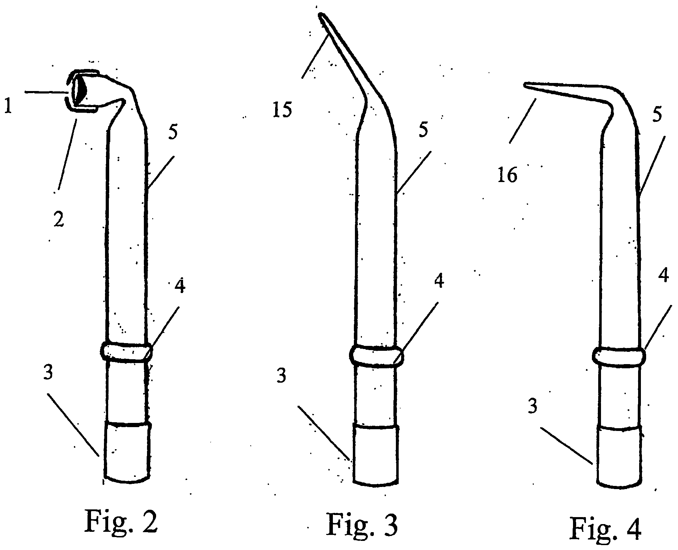

   Figur 2 zeigt eine bevorzugte Ausführungsform der erfindungsgemäßen Behandlungssonde, eine Kariessonde.
**Figuren 3 bis 8**
   Die Figuren 3 bis 8 zeigen bevorzugte Ausführungsformen der erfindungsgemäßen Behandlungssonden.
**Figur 9**

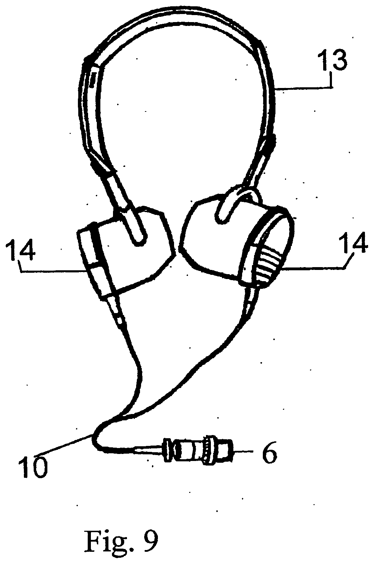

   Figur 9 zeigt das erfindungsgemäße Zusatzgerät für das erfindungsgemäße Behandlungsgerät, bei dem die Vorrichtung, die ein elektromagnetisches Feld bildet, jeweils an den zwei Enden eines offenen kreisförmigen Bügels angeordnet ist.
**Figur 10**

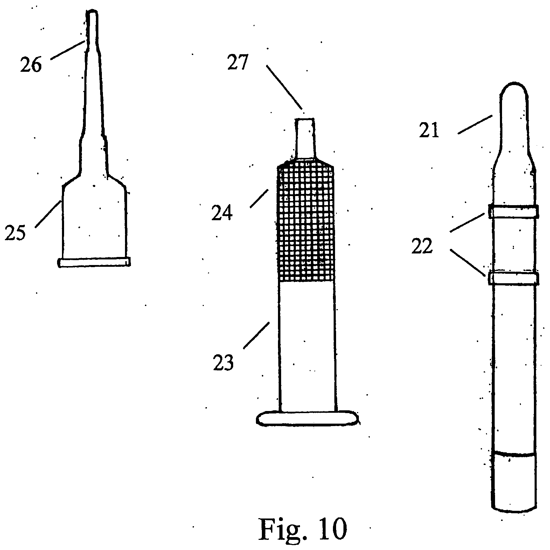

   Figur 10 zeigt eine Vorrichtung, bei der auf die erfindungsgemäße Behandlungssonde eine Gasaufnahmevorrichtung geschoben ist.
**Figur 11**

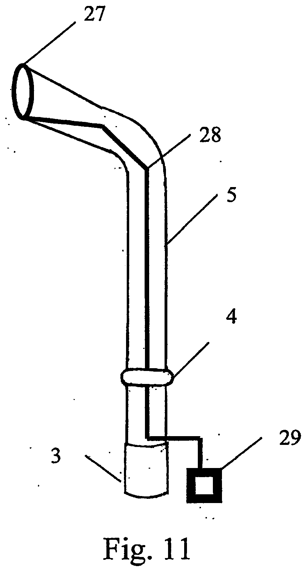

   In Figur 11 wird eine bevorzugte Ausführungsform der erfindungsgemäßen Behandlungssonde mit der stromableitenden Vorrichtung dargestellt.
**Figur 12**

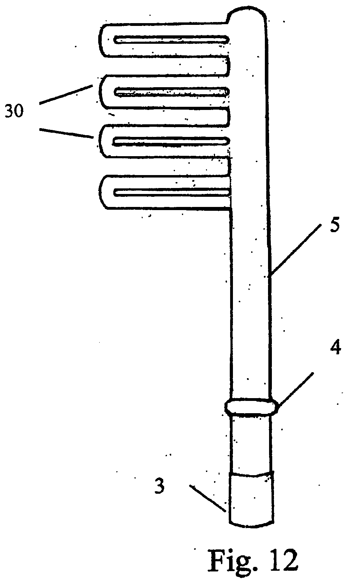

   Figur 12 zeigt eine Kammsonde.

In **Figur 1** wird eine bevorzugte Ausführungsform des erfindungsgemäßen Behandlungsgeräts dargestellt. Das Behandlungsgerät weist einen von den bauartbeding relativ klein gebauten Grundkörper (6) auf, an dem ein Drehknopf (7) angebracht ist, mit dem die wirksame Menge des oxidierenden Gases eingestellt wird sowie Indikationsfelder (8) mit denen die wirksame Menge des oxidierenden Gases angezeigt wird. An dem Grundkörper ist über eine Anschlußkupplung (9) ein Spiralkabel (10) angebracht, das zum Handstück (11) führt und Spulen (nicht gezeigt) enthält. Am Ende des Handstücks (11) ist die, je nach Therapie, jeweilige Sonde (12) angebracht.

**Figur 2** zeigt eine CA-Behandlungssonde für die Behandlung von Karries, die an ihrer Spitze ein vorzugsweise konkaves Ende (1) mit vorzugsweise einer Kappe (2) als Abstandshalter, vorzugsweise aus Silicon ist, aufweist, die bis zu 300.000 ppm atomaren Sauerstoff ausschließlich in der Kavität erzeugt. Des weiteren kann auch eine Kappe aufgesetzt werden, die in einer Kapillare endet oder eine Kappe die über den Zahn übergreift. Die CA-Behandlungssonde kann auch ohne die Kappe betrieben werden, die als Abstandshalter dient. Das untere Ende der Sonde ist ein zylinderförmiger Metallverschluss (3), wobei die Sonde einen hohlen Glasgrundkörper (5) aufweist, der einen Gummiring (4) aufweist, der verhindert, dass Flüssigkeiten an der Behandlungssonde entlang bis ins Innere des Handgriffs dringt, in dem die Behandlungssonde formschlüssig gesteckt wird und so die Kontaktfläche zwischen dem unteren Metallverschluß am Ende der Behandlungssonde und dem Hangriff verschmutzen könnte, die schwer zu reinigen ist.

**Figur 3** zeigt eine Behandlungssonde, die eine PA/8-Sonde für Pa Bereich 8-er ist, die an ihrer Spitze (15) eine feine Spitze im Winkel von 45°C aufweist, wobei die Spitze auch vorzugsweise konvex ausgebildet sein kann. Der Grundkörper ist wie bei der Behandlungssonde in Figur 2 beschrieben.

**Figur 4** zeigt eine Behandlungssonde, die eine PA-Sonde für Parodontitis ist, die eine feine Spitze (16) im Winkel von 90°C aufweist, wobei die Spitze auch vorzugsweise konvex ausgebildet sein kann. Der Grundkörper ist wie bei der Behandlungssonde in Figur 2 beschrieben.

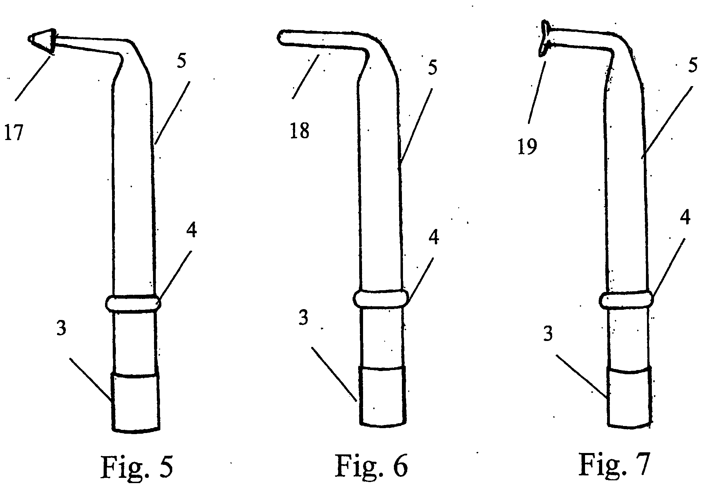

**Figur 5** zeigt eine Behandlungssonde, die eine CR-Sonde für vorzugsweise die Wurzelkanalbehandlung, Fisuren oder Karies ist, die an ihrer Spitze (17) ein kegelförmiges Ende aufweist, wobei das kegelförmige Ende auch dadurch gebildet werden kann, dass auf eine Behandlungssonde nach Figur 3 ein Kegel aus Kunststoff gesteckt wird. Der Kegel dient dazu einen Abstand zum Wurzelkanal zu halten, insbesondere um die Bruchgefahr der Glassonde zu vermindern. Die Spitze kann auch vorzugsweise konvex ausgebildet sein. Der Grundkörper ist wie bei der Behandlungssonde in Figur 2 beschrieben.

**Figur 6** zeigt eine Behandlungssonde, die eine AL-Sonde für die Alveole und einen Fräskanal ist, die an ihrer Spitze (18) eine stumpfe, gegebenenfalls kugelförmige, vorzugsweise konvex ausgebildete Spitze aufweist. Der Grundkörper ist wie bei der Behandlungssonde in Figur 2 beschrieben.

**Figur 7** zeigt eine Behandlungssonde, die eine GI-Sonde für die Gingiva ist, die an ihrer Spitze ein sich erweiterndes pilzförmiges, vorzugsweise konkaves Ende (19) aufweist. Der Grundkörper ist wie bei der Behandlungssonde in Figur 2 beschrieben.

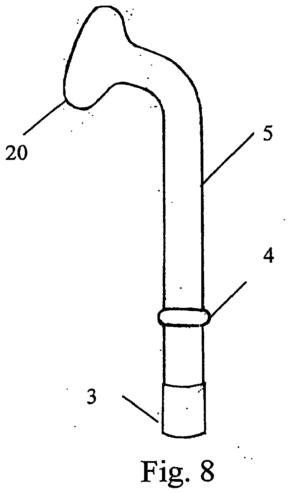

**Figur 8** ist eine Behandlungssonde vorzugsweise für die Behandlung der Hautoberfläche bei infektiösen oder entzündeten Wunden und weist eine extra große linsenförmiges, ebenes oder auch konvexes oder konkaves Ende (20) auf. Der Grundkörper ist wie bei der Behandlungssonde in Figur 2 beschrieben.

**Figur 9** zeigt das erfindungsgemäße Zusatzgerät für das erfindungsgemäße Behandlungsgerät, bei dem die Vorrichtung, die ein elektromagnetisches Feld bildet, jeweils an den zwei Enden (14) eines offenen kreisförmigen Bügels (13) angeordnet ist und ein Kabel (9) mit Anschlußkupplung (10) zum Anschließen an das erfindungsgemäße Behandlungsgerät (9) aus Figur 1.

**Figur 10** zeigt ein erfindungsgemäßes Zusatzgerät für die prinzipiell jede der erfindungsgemäßen Behandlungssonden verwendet werden kann, wobei auf die Sonde eine Gasaufnahmevorrichtung (23) aufgebracht wird. Diese Gasaufnahmevorrichtung (23) kann eine übliche Spritze (23) aus Kunststoff, Glas, isoliertes Metall oder auch Metall sein. Diese wird vorzugsweise gasdicht auf die Sondenspitze (21) aufgesetzt, wobei die Sonde selbst noch Dichtringe (22) aufweisen kann, um besser abzudichten. Die erfindungsgemäße Gasaufnahmevorrichtung (23) läßt sich vorzugsweise entlang der Längsachse der Sonde verschieben, um das darin befindliche erzeugte oxidierende Gas aus der Gasaufnahmevorrichtung (23) zu befördern. Dies geschieht über eine Öffnung, die offen ist oder geschlossen werden kann und an der ein Aufsatz mit einer Kanüle (25) und einer Kapillare (26), vorzugsweise aus Kunststoff oder Metall, aufgesetzt wird, die flexibel oder nicht flexible ist, über die das Gas in eine Wundtasche am Zahn, den Wurzelkanal oder zur Wurzelresektion oder an sonstige Applikationsstellen eingeführt wird, wo eine Entkeimung stattfinden soll. Das oxidierende Gas wird aus dem Luftsauerstoff gebildet, indem durch den Betrieb der Behandlungssonde ein elektrisches Feld erzeugt wird. Der hierzu benötigte Sauerstoff wird über den Kolben aufgezogen oder mit einer Schlauchleitung extern zugeführt.

In der Gasaufnahmevorrichtung (23) kann sich ein Metalleinsatz (24) befinden, der durch ein oxidierendes Gas nicht angegriffen wird, wie Edelstahl, Titan, einem Edelmetall, wie Platin, Rhodium, Gold etc. befinden, das die Erzeugung des oxidierenden Gases verbessert, indem mit dem Finger darauf gedrückt wird, um die Gegenelektrode zu bilden. Bei dem Metalleinsatz kann es sich um einen Stift, ein Rohr oder bevorzugt ein Gitter handeln. Prinzipiell kann das oxidierende Gas auch ohne Metalleinsatz nur durch Drücken mit einem Finger auf den vorderen Teil der Gasaufnahmevorrichtung (23) gebildet werden.

**Figur 11** ist eine Behandlungssonde, die die erfindungsgemäße stromableitende Vorrichtung aufweist, wobei diese eine Befestigungsvorrichtung (27) für vorzugsweise einen Draht (28) der zur Masse (29) geführt wird, aufweist. Der Grundkörper ist ein hohler Glasgrundkörper (5), der einen Gummiring (4) aufweist, der verhindert, dass Flüssigkeiten an der Behandlungssonde entlang bis ins Innere des Handgriffs dringt, in dem die Behandlungssonde formschlüssig gesteckt wird und so die Kontaktfläche zwischen dem unteren Metallverschluß am Ende der Behandlungssonde und dem Hangriff verschmutzen könnte, die schwer zu reinigen ist.

**Figur 12** zeigt eine Kammsonde, die eine Sonde ist, um Erkrankungen der Kopfhaut oder der Haarfolikel zu behandeln und die Form eines Kamm aufweists, wobei die Zinken (30) des Kammes in diesem Fall U-förmige Glasrohre sind, die mit dem Grundkörper der Sonde so verbunden sind, dass die U-förmigen Röhrchen offen zum Grundkörper der Sonde sind, so dass das Gas ungehindert in der gesamten Sonde zirkulieren kann. Der Grundkörper ist wie bei der Behandlungssonde in Figur 2 beschrieben.

## Patentansprüche

1. Behandlungsgerät, das eine Vorrichtung aufweist, die ein elektrisches oder elektromagnetisches Feld erzeugt, sich im wesentlichen in einem Handgriff (11) befindet, der seriell geschaltete Spulen aufweist und eine hohle Glassonde (12) aufweist, die mit zumindest einem elektrisch leitenden Gas gefüllt ist, wobei das elektrische oder elektromagnetische Feld zwischen einer Sonde und dem Körper eines Menschen oder Tieres oder einem Metall gebildet wird, **dadurch gekennzeichnet, dass** die Feldspannung und Stromstärke innerhalb des elektrischen oder elektromagnetischen Feldes erst bei Annäherung an den Körper oder das Metall, von einer niedrigen Feldspannung und Stromstärke auf eine höhere Feldspannung und Stromstärke ansteigt, wobei bei der niedrigen Feldspannung und Stromstärke ein minimaler Strom fließt, wobei es zu einer Entladung kommt, die einen Impuls auslöst, so dass die Stromstärke innerhalb einer Zeiteinheit auf eine höhere Stromstärke ansteigt.

2. Behandlungsgerät nach Anspruch 1, **dadurch gekennzeichnet, dass** nach dem Anstieg der Feldspannung und Stromstärke des elektrischen oder elektromagnetischen Feldes dieses innerhalb einer Zeiteinheit wieder abfällt, wobei sich der Vorgang Anstieg und Abfall der Feldspannung und Stromstärke wiederholt.

3. Behandlungsgerät nach Anspruch 1 oder 2, **dadurch gekennzeichnet, dass** die Zeiteinheit 0,25 bis 10 Sekunde beträgt.

4. Behandlungsgerät nach einem oder mehreren der Ansprüche 1 bis 3, **dadurch gekennzeichnet, dass** das Gerät eine Schnittstelle aufweist über die alle Einstellungen in dem Gerät von außen veränderbar sind.

5. Behandlungsgerät nach einem oder mehreren der Ansprüche 1 bis 4, **dadurch gekennzeichnet, dass** das Gerät oxidierendes Gas über eine Gasleitung liefert.

6. Behandlungsgerät nach Anspruch 5, **dadurch gekennzeichnet, dass** die Gaszuleitung zu einen Formkörper führt, der sich an die Anatomie eines Menschen oder Tieres anpaßt.

7. Behandlungsgerät nach einem oder mehreren der Ansprüche 1 bis 6, **dadurch gekennzeichnet, dass** es eine Vorrichtung aufweist, die das elektrische oder elektromagnetische Feld mit einer Feldspannung von 1.800 bis 35.000 V mittels einer Spannung von 12 bis 600 V, einer Stromstärke von 0,1 µA bis 100 µA und einer Frequenz von 10.000 bis 35.000 Hz erzeugt.

8. Behandlungsgerät nach einem oder mehreren der Ansprüche 1 bis 7, **dadurch gekennzeichnet, dass** es eine Vorrichtung aufweist, die das elektrische oder elektromagnetische Feld mit einer Spannung von 12 V bis 50 V, besonders bevorzugt von 18 V bis 28 V erzeugt.

9. Behandlungsgerät nach einem oder mehreren der Ansprüche 1 bis 8, **dadurch gekennzeichnet, dass** es eine Vorrichtung aufweist, die das elektrische oder elektromagnetische Feld mit einer Stromstärke von 0,1 µA bis 20 µA, besonders bevorzugt mit einer Stromstärke von 0,8 µA bis 10 µA erzeugt.

10. Behandlungsgerät nach einem oder mehreren der Ansprüche 1 bis 9, **dadurch gekennzeichnet, dass** die Vorrichtung, die ein elektrisches oder elektromagnetisches Feld bildet, sich im wesentlichen in einem Handgriff (11) befindet, der seriell geschaltete Spulen aufweist und eine hohle Glassonde (12) aufweist, die mit zumindest einem Edelgas gefüllt ist.

11. Behandlungsgerät nach einem oder mehreren der Ansprüche 1 bis 10, **dadurch gekennzeichnet, dass** die Vorrichtung, die ein elektrisches oder elektromagnetisches Feld bildet, jeweils an den zwei Enden (14) eines offenen kreisförmigen Bügels (13) angeordnet ist.

12. Behandlungsgerät nach einem oder mehreren der Ansprüche 1 bis 11, **dadurch gekennzeichnet, dass** die Vorrichtung, die ein elektrisches oder elektromagnetisches Feld bildet, als Behandlungssonde eine hohle Glassonde (12, 5), die mit zumindest einem elektrisch leitenden Gas gefüllt ist, aufweist.

13. Behandlungsgerät nach einem oder mehreren der Ansprüche 1 bis 12, **dadurch gekennzeichnet, dass** die Behandlungssonde (12, 5) eine stromableitende Vorrichtung zur Erdung (27, 28, 29) aufweist.

14. Behandlungsgerät nach einem oder mehreren der Ansprüche 1 bis 13, **dadurch gekennzeichnet, dass** die Behandlungssonde (12, 5) mit zumindest einem Edelgas oder einem beliebigen Edelgasgemisch gefüllt ist.

15. Behandlungsgerät nach einem oder mehreren der Ansprüche 1 bis 14, **dadurch gekennzeichnet, dass** die Behandlungssonde (12, 5) mit einem Edelgasgemisch aus Argon und Neon gefüllt ist.

16. Behandlungsgerät nach einem oder mehreren der Ansprüche 1 bis 15, **dadurch gekennzeichnet, dass** die Behandlungssonde (12, 5) mit einem Edelgasgemisch aus 30 Vol.% Argon und 70 Vol.% Neon gefüllt ist.

17. Behandlungsgerät nach einem oder mehreren der Ansprüche 1 bis 16, **dadurch gekennzeichnet, dass** die Behandlungssonde (12, 5) an ihrem oberen Ende als eine feine Spitze (15, 16), gegebenenfalls konvex, ausgebildet ist.

18. Behandlungsgerät nach einem oder mehreren der Ansprüche 1 bis 17, **dadurch gekennzeichnet, dass** die Behandlungssonde (12, 5) an ihrem oberen Ende als kegelförmige Spitze (17), gegebenenfalls konvex, ausgebildet ist.

19. Behandlungsgerät nach einem oder mehreren der Ansprüche 1 bis 18, **dadurch gekennzeichnet, dass** die Behandlungssonde (12, 5) an ihrem oberen Ende als stumpfes, gegebenenfalls kugelförmiges Ende (18), gegebenenfalls konvex oder konkav, ausgebildet ist.

20. Behandlungsgerät nach einem oder mehreren der Ansprüche 1 bis 19, **dadurch gekennzeichnet, dass** die Behandlungssonde (12, 5) an ihrem oberen Ende als linsenförmiges Ende (19, 20) gegebenenfalls konvex oder konkav, ausgebildet ist.

21. Behandlungsgerät nach einem oder mehreren der Ansprüche 1 bis 20, **dadurch gekennzeichnet, dass** die Behandlungssonde (12, 5) an ihrem oberen Ende als konkaves Ende (1) ausgebildet ist.

22. Behandlungsgerät nach einem oder mehreren der Ansprüche 1 bis 21, **dadurch gekennzeichnet, dass** die Behandlungssonde (12, 5) an ihrem oberen Ende als erweitertes Ende ausgebildet ist.

23. Behandlungsgerät nach einem oder mehreren der Ansprüche 1 bis 22, **dadurch gekennzeichnet, dass** die Behandlungssonde (12, 5) an ihrem oberen Ende eine elastische Kappe (2) aufweist, die an ihrem oberen Ende offen ist.

24. Behandlungsgerät nach einem oder mehreren der Ansprüche 1 bis 23, **dadurch gekennzeichnet, dass** die Behandlungssonde (12, 5) jeweils an den zwei Enden (14) eines offenen kreisförmigen Bügels (13) angeordnet ist.

25. Behandlungsgerät nach einem oder mehreren der Ansprüche 12 bis 22, **dadurch gekennzeichnet, dass** sich auf der Sonde eine Gasaufnahmevorrichtung (23) befindet.

26. Behandlungsgerät nach Anspruch 25 , **dadurch gekennzeichnet, dass** die Gasaufnahmevorrichtung (23) auf der Sonde sich entlang der Längsachse der Sonde verschieben läßt.

27. Behandlungsgerät nach Anspruch 25, **dadurch gekennzeichnet, dass** die Gasaufnahmevorrichtung (23) auf der Sonde einen Metalleinsatz (24) aufweist.

28. Behandlungsgerät nach Anspruch 25, **dadurch gekennzeichnet, dass** die Gasaufnahmevorrichtung (23) auf der Sonde an einem Ende eine Öffnung (27) aufweist, die offen ist oder geschlossen werden kann.

29. Behandlungsgerät nach Anspruch 25, **dadurch gekennzeichnet, dass** sich an die Öffnung (27) der Gasaufnahmevorrichtung (23) auf der Sonde eine Kanüle (25) anschließt, die eine flexible oer nicht flexible Kapillare (26) aufweist.

## Claims

1. A Treatment appliance comprising a device for generating an electrical or electromagnetic field substantially present in a handle (11) comprising coils connected in series and comprising a hollow glass probe (12) filled with at least one electrically conductive gas, the electrical or electromagnetic field being formed between a probe and the body of a person or an animal or a metal, **characterized in that** the field voltage and current intensity within the electrical or electromagnetic field increases from a low field voltage and current intensity to a higher field voltage and current intensity only when approaching the body or the metal, wherein a minimal current flows at the low field voltage and current intensity, wherein a discharge occurs and initiates an impulse, so that the current intensity increases to a higher current intensity within a unit of time.

2. The treatment appliance according to claim 1, **characterized in that** after the increase in field voltage and current intensity of the electrical or electromagnetic field, said field decreases again within a unit of time, wherein the sequence of increase and decrease of the field voltage and current intensity repeats itself.

3. The treatment appliance according to claim 1 or 2, **characterized in that** the unit of time is 0.25 to 10 *seconds.

4. The treatment appliance according to any one or more of the claims 1 through 3, **characterized in that** the device comprises an interface by means of which all settings in the device can be modified from the outside.

5. The treatment appliance according to any one or more of the claims 1 through 4, **characterized in that** the device provides oxidizing gas by means of a gas line.

6. The treatment appliance according to claim 5, **characterized in that** the gas infeed line leads to a shaped body adapting to the anatomy of a person or animal.

7. The treatment appliance according to any one or more of the claims 1 through 6, **characterized in that** said device comprises a device for generating the electrical or electromagnetic field having a field voltage from 1,800 to 35,000 V by means of a voltage from 12 to 600 V, a current intensity from 0.1 µA to 100 µA, and a frequency from 10,000 to 35,000 Hz.

8. The treatment appliance according to any one or more of the claims 1 through 7, **characterized in that** said device comprises a device for generating the electrical or electromagnetic field having a voltage from 12 V to 50 V, particularly preferably from 18 V to 28 V.

9. The treatment appliance according to any one or more of the claims 1 through 8, **characterized in that** said appliance comprises a device for generating the electrical or electromagnetic field having a current intensity from 0.1 µA to 20 µA, particularly preferably having a current intensity from 0.8 µA to 10 µA.

10. The treatment appliance according to any one or more of the claims 1 through 9, **characterized in that** the device forming an electrical or electromagnetic field is substantially present in a handle (11) comprising coils connected in series and comprising a hollow glass probe (12) filled with at least one noble gas.

11. The treatment appliance according to any one or more of the claims 1 through 10, **characterized in that** the device forming an electrical or electromagnetic field is disposed at each of the two ends (14) of an open, circular hoop (13).

12. The treatment appliance according to any one or more of the claims 1 through 11, **characterized in that** the device forming an electrical or electromagnetic field comprises as a treatment probe a hollow glass probe (12, 5) filled with at least one electrically conductive gas.

13. The treatment appliance according to any one or more of the claims 1 through 12, **characterized in that** the treatment probe (12, 5) comprises a current-conducting device for grounding (27, 28, 29).

14. The treatment appliance according to any one or more of the claims 1 through 13, **characterized in that** the treatment probe (12, 5) is filled with at least one noble gas or an arbitrary mixture of noble gases.

15. The treatment appliance according to any one or more of the claims 1 through 14, **characterized in that** the treatment probe (12, 5) is filled with a noble gas mixture of argon and neon.

16. The treatment appliance according to any one or more of the claims 1 through 15, **characterized in that** the treatment probe (12, 5) is filled with a noble gas mixture of 30% argon by volume and 70% neon by volume.

17. The treatment appliance according to any one or more of the claims 1 through 16, **characterized in that** the treatment probe (12, 5) is implemented as an optionally convex fine point (15, 16) at the top end thereof.

18. The treatment appliance according to any one or more of the claims 1 through 17, **characterized in that** the treatment probe (12, 5) is implemented as an optionally convex conical point (17) at the top end thereof.

19. The treatment appliance according to any one or more of the claims 1 through 18, **characterized in that** the treatment probe (12, 5) is implemented as a blunt, optionally spherical, optionally convex or concave end (18) at the top end thereof.

20. The treatment appliance according to any one or more of the claims 1 through 19, **characterized in that** the treatment probe (12, 5) is implemented as a lens-shaped, optionally convex or concave end (19, 20) at the top end thereof.

21. The treatment appliance according to any one or more of the claims 1 through 20, **characterized in that** the treatment probe (12, 5) is implemented as a concave end (1) at the top end thereof.

22. The treatment appliance according to any one or more of the claims 1 through 21, **characterized in that** the treatment probe (12, 5) is implemented as an extended end at the top end thereof.

23. The treatment appliance according to any one or more of the claims 1 through 22, **characterized in that** the treatment probe (12, 5) comprises at the top end thereof an elastic cap (2) open at the top end thereof.

24. The treatment appliance according to any one or more of the claims 1 through 23, **characterized in that** the treatment probe (12, 5) is disposed at each of the two ends (14) of an open, circular hoop (13).

25. The treatment appliance according to any one or more of the claims 12 through 22, **characterized in that** a gas receiving device (23) is present on the probe.

26. The treatment appliance according to claim 25, **characterized in that** the gas receiving device (23) on the probe can be displaced along the longitudinal axis of the probe.

27. The treatment appliance according to claim 25, **characterized in that** the gas receiving device (23) on the probe comprises a metal insert (24).

28. The treatment appliance according to claim 25, **characterized in that** the gas receiving device (23) on the probe comprises an opening at one end, said opening being open or being closable.

29. The treatment appliance according to claim 25, **characterized in that** a cannula (25) connects to the opening (27) of the gas receiving device (23) on the probe and comprises a flexible or non-flexible capillary (26).

## Revendications

1. Appareil de traitement comprenant un dispositif qui génère un champ électrique ou électromagnétique, qui est situé essentiellement dans une poignée (11) comportant des bobines couplées en série et une sonde en verre (12) creuse qui est remplie d'au moins un gaz électroconducteur, sachant que le champ électrique ou électromagnétique est formé entre une sonde et le corps d'un être humain ou d'un animal ou un métal, **caractérisé en ce que** la tension de champ et l'intensité du courant dans le champ électrique ou électromagnétique n'augmentent d'une tension de champ et d'une intensité de courant faibles à une tension de champ et une intensité de courant plus élevées qu'à l'approche vers le corps ou le métal, sachant qu'un courant minimal circule à la tension de champ et à l'intensité de courant faibles, sachant qu'il se produit une décharge qui déclenche une impulsion, de sorte que l'intensité du courant augmente à une intensité de courant plus élevée dans une unité de temps.

2. Appareil de traitement selon la revendication 1, **caractérisé en ce que**, après l'augmentation de la tension de champ et de l'intensité de courant du champ électrique ou électromagnétique, celui-ci chute à nouveau dans une unité de temps, sachant que le processus augmentation et chute de la tension de champ et de l'intensité de courant se répète.

3. Appareil de traitement selon la revendication 1 ou 2, **caractérisé en ce que** l'unité de temps est de 0,25 à 10 secondes.

4. Appareil de traitement selon l'une quelconque ou plusieurs des revendications 1 à 3, **caractérisé en ce que** l'appareil présente une interface via laquelle tous les réglages de l'appareil peuvent être modifiés de l'extérieur.

5. Appareil de traitement selon l'une quelconque ou plusieurs des revendications 1 à 4, **caractérisé en ce que** l'appareil fournit un gaz oxydant via une conduite de gaz.

6. Appareil de traitement selon la revendication 5, **caractérisé en ce que** l'arrivée de gaz conduit à un corps formé qui s'adapte à l'anatomie d'un être humain ou d'un animal.

7. Appareil de traitement selon l'une quelconque ou plusieurs des revendications 1 à 6, **caractérisé en ce qu'**il comprend un dispositif qui génère le champ électrique ou électromagnétique avec une tension de champ de 1 800 à 35000 V au moyen d'une tension de 12 à 600 V, une intensité de courant de 0,1 µA à 100 µA et une fréquence de 10 000 à 35 000 Hz.

8. Appareil de traitement selon l'une quelconque ou plusieurs des revendications 1 à 7, **caractérisé en ce qu'**il comprend un dispositif qui génère le champ électrique ou électromagnétique avec une tension de 12 V à 50 V, de manière particulièrement préférée de 18 V à 28 V.

9. Appareil de traitement selon l'une quelconque ou plusieurs des revendications 1 à 8, **caractérisé en ce qu'**il comprend un dispositif qui génère le champ électrique ou électromagnétique avec une intensité de courant de 0,1 µA à 20 µA, de manière particulièrement préférée de 0,8 µA à 10 µA.

10. Appareil de traitement selon l'une quelconque ou plusieurs des revendications 1 à 9, **caractérisé en ce que** le dispositif qui forme un champ électrique ou électromagnétique est essentiellement situé dans une poignée (11) qui comprend des bobines couplées en série et une sonde en verre (12) creuse qui est remplie d'au moins un gaz rare.

11. Appareil de traitement selon l'une quelconque ou plusieurs des revendications 1 à 10, **caractérisé en ce que** le dispositif qui forme un champ électrique ou électromagnétique est disposé à chacune des deux extrémités (14) d'un étrier (13) circulaire ouvert.

12. Appareil de traitement selon l'une quelconque ou plusieurs des revendications 1 à 11, **caractérisé en ce que** le dispositif qui forme un champ électrique ou électromagnétique comporte en tant que sonde de traitement une sonde en verre (12, 5) creuse qui est remplie d'au moins un gaz électroconducteur.

13. Appareil de traitement selon l'une quelconque ou plusieurs des revendications 1 à 12, **caractérisé en ce que** la sonde de traitement (12, 5) présente un dispositif de dérivation de courant pour la mise à la terre (27, 28, 29).

14. Appareil de traitement selon l'une quelconque ou plusieurs des revendications 1 à 13, **caractérisé en ce que** la sonde de traitement (12, 5) est remplie d'au moins un gaz rare ou d'un quelconque mélange de gaz rares.

15. Appareil de traitement selon l'une quelconque ou plusieurs des revendications 1 à 14, **caractérisé en ce que** la sonde de traitement (12, 5) est remplie d'un mélange de gaz rares composé d'argon et de néon.

16. Appareil de traitement selon l'une quelconque ou plusieurs des revendications 1 à 15, **caractérisé en ce que** la sonde de traitement (12, 5) est remplie d'un mélange de gaz rares composé de 30 % en volume d'argon et de 70 % en volume de néon.

17. Appareil de traitement selon l'une quelconque ou plusieurs des revendications 1 à 16, **caractérisé en ce que** la sonde de traitement (12, 5) se présente à son extrémité supérieure sous la forme d'une pointe (15, 16) fine, le cas échéant convexe.

18. Appareil de traitement selon l'une quelconque ou plusieurs des revendications 1 à 17, **caractérisé en ce que** la sonde de traitement (12, 5) se présente à son extrémité supérieure sous la forme d'une pointe (17) conique, le cas échéant, convexe.

19. Appareil de traitement selon l'une quelconque ou plusieurs des revendications 1 à 18, **caractérisé en ce que** la sonde de traitement (12, 5) se présente à son extrémité supérieure sous la forme d'une extrémité (18) contondante, le cas échéant sphérique, le cas échéant convexe ou concave.

20. Appareil de traitement selon l'une quelconque ou plusieurs des revendications 1 à 19, **caractérisé en ce que** la sonde de traitement (12, 5) se présente à son extrémité supérieure sous la forme d'une extrémité (19, 20) lenticulaire, le cas échéant convexe ou concave.

21. Appareil de traitement selon l'une quelconque ou plusieurs des revendications 1 à 20, **caractérisé en ce que** la sonde de traitement (12, 5) se présente à son extrémité supérieure sous la forme d'une extrémité (1) concave.

22. Appareil de traitement selon l'une quelconque ou plusieurs des revendications 1 à 21, **caractérisé en ce que** la sonde de traitement (12, 5) se présente à son extrémité supérieure sous la forme d'une extrémité élargie.

23. Appareil de traitement selon l'une quelconque ou plusieurs des revendications 1 à 22, **caractérisé en ce que** la sonde de traitement (12, 5) comporte à son extrémité supérieure un capuchon élastique (2) qui est ouvert à son extrémité supérieure.

24. Appareil de traitement selon l'une quelconque ou plusieurs des revendications 1 à 23, **caractérisé en ce que** la sonde de traitement (12, 5) est disposée à chacune des deux extrémités (14) d'un étrier (13) circulaire ouvert.

25. Appareil de traitement selon l'une quelconque ou plusieurs des revendications 12 à 22, **caractérisé en ce qu'**un dispositif (23) d'aspiration de gaz est disposé sur la sonde.

26. Appareil de traitement selon la revendication 25, **caractérisé en ce que** le dispositif (23) d'aspiration de gaz sur la sonde peut être déplacé le long de l'axe longitudinal de la sonde.

27. Appareil de traitement selon la revendication 25, **caractérisé en ce que** le dispositif (23) d'aspiration de gaz sur la sonde comporte un insert métallique (24).

28. Appareil de traitement selon la revendication 25, **caractérisé en ce que** le dispositif (23) d'aspiration de gaz sur la sonde présente à une extrémité une ouverture (27) qui est ouverte ou peut être fermée.

29. Appareil de traitement selon la revendication 25, **caractérisé en ce que** l'ouverture (27) du dispositif (23) d'aspiration de gaz sur la sonde est suivie d'une canule (25) qui présente un capillaire (26) flexible ou non flexible.
